# EUROPEAN PATENT APPLICATION

(11) **EP 4 342 977 A1**
(43) Date of publication of application: **27.03.2024**
(21) Application number: 22197851.3
(22) Date of filing: 26.09.2022
(51) Int. Cl.: C12N 5/00, C12N 5/071

(54) **SERUM FREE MEDIUM**

(71) Applicant: Ares Trading S.A., 1170 Aubonne, VD (CH)
(72) Inventor: NEVELLI, Francesco, 10010 Colleretto Giacosa (TO) (IT); VIGNA, Virginia, 10010 Colleretto Giacosa (TO) (IT)
(74) Representative: Merck Serono S.A. Intellectual Property

(57) **Abstract**

Provided herein is a cell culture serum free medium for culturing cells in-vitro, and methods of using the cell culture serum free medium.

## Description

### Field of the Invention

The present invention relates to media and solutions for cell growth of cultured cells.

### Background.

In-vitro cell-based assays are widely used to characterize a molecule's bioactivity or quantify the potency of clinical and marketed drugs, as requested by regulatory authorities [1]. To evaluate the drug potency, the assays should reflect or mimic the product's known or intended mechanism of action as much as possible [1]. For this reason, cell-based bioassays are performed using cell lines that respond to specific ligands or infectious agents producing a signal easily measurable [1].

Fetal Bovine Serum (FBS) is the gold standard of cell supplements, and it is commonly used to support cell growth in-vitro. However, it is one of the most critical reagents for cell culture for different reasons [2].

Firstly, FBS use implies significant ethical issues since its production causes suffering to animals. Secondly, FBS is one of the higher sources of cell culture contamination. Finally, FBS composition is not completely characterized, and this could lead to batch-to-batch variability and could impact the analytical precision. For all these reasons, pharmaceutical companies are committed to removing FBS from different processes, since its elimination appears as a safe, cost-effective, and ethical solution [2].

### Summary of the Invention.

The present invention provides a cell culture serum free medium for use in in-vitro cell culture processes and procedures. The serum free medium for culturing cells comprises a) Dulbecco's Modified Eagle's Medium (DMEM), b) a nutrient mix, c) an antioxidant and d) a supplemental source of glutamine. Preferably, the nutrient mix is nutrient mixture F-12 Ham. Furthermore, preferred is that the antioxidant is 2-mercaptoethanol. The supplemental glutamine source may be selected from glutamine or GlutaMAX.

### Brief Description of the Drawings

Figure 1 shows a description of the four adaptation protocols to SFM.
Figure 2 shows cell viability measured during the whole HEK-293T cell adaptation process. 2A) All protocols with SFM_A and 2B) All protocols with SFM_ B.
Figure 3 shows HEK 293-T viability whit the different adaptation protocols using SFM_A. The red line represents a 75% viability threshold.
Figure 4. Shows images of HEK-293T cells in SFM type A acquired with Incucyte^{®} to identify the cell confluence in each protocol at passage 11. Magnification: 10x.
Figure 5. The cell growth rate in different serum-free conditions seeded at the same concentrations: RED: HEK-293T, YELLOW: Protocol 1, GREEN: Protocol2 and BLUE: Protocol 3.

### Detailed Description of the Invention.

In recent years, medium supplements are becoming an important role in cell adaptation and proliferation. In fact, the receptors involved in cell survival, growth, and differentiation are different in the cells and release different factors into their environment. To develop a homemade SFM specific for HEK-293T cells, initial scouting of culture media components was performed. The first component for our homemade SFM was the basal medium that contained a mixture of 50% of DMEM and 50% of F-12. The combination of the two media ensures a high amino acid content needed for cell survival and growth. The basal medium is then supplemented with a solution of Insulin-Transferrin-Selenium (ITS). Insulin is known to be essential in cell culture for cell growth and metabolism. Transferrin is an iron carrier, and it helps reduce the toxic levels of oxygen radicals and peroxide produced during cell culture. Selenium protects cells against oxidative stress reducing the production of free radicals [3].

Another important component in SFM is glutamine, an essential precursor for the synthesis of proteins and ribonucleotides. However, glutamine used in SFM could have a negative effect due to its instability in solution. In fact, serum proteins avoid glutamine's breakdown and metabolism that cause the production and accumulation of ammonia, which is toxic to cells. An alternative reagent to glutamine, is Glutamax which is more stable and resistant. To verify the best component, two different SFM are developed where SFM_A contains Glutamax and SFM_B contain glutamine. Finally, b-mercaptoethanol is added to the formulation in order to protect cells from oxidative stress in culture. These two media will be used as essential media to test HEK-293T adaptation in serum-free culture.

However, additional components could be used to better support cell survival, proliferation, and adhesion. Literature suggested the use of specific hormones and growth factors such as epidermal growth factor (EGF) and glucocorticoids (hydrocortisone and dexamethasone), lipids, and vitamins [3]. All these components promote different cell functions such as proliferation, differentiation, and cell migration. In addition to them, some matrix components such as fibronectin or vitronectin could be added to maintain cell adhesion.

While as shown herein the serum free medium was developed using HEK 293 cells, the medium can be used for various cells lines, in particularly those cell lines which under conventional circumstances would grown in similar growth media as HEK 293 cells.

### Examples:

### Cell line

HEK-293T (Creative Bioarray, NY, USA), a human embryonic kidney cell line, is cultured in flasks and 6-wells plates. Cells used as controls are maintained in culture according to the supplier's instructions. For cells were cultured with the various adaptation protocols. Cell number and viability are assessed using NucleoCounter^{®} (Chemometec, Denmark).

### Serum-containing medium (SCM)

HEK-293T are cultured in the medium suggested by the supplier: Dulbecco's Modified Eagle's Medium (DMEM) (Merck Life Science, MO, USA) supplemented with 10% FBS (Thermo Fisher, MA, USA) and 1.5% Penicillin-Streptomycin (Thermo Fisher, MA, USA). Cells are maintained in an incubator at 37.0°C in a humidified atmosphere with 5.0±1.0% CO2.

### Serum-free media (SFM)

Two serum-free culture media are used during the adaptation process: Serum-Free Medium type A (SFM_A) and Serum-Free Medium type B (SFM_B). Both media contain: Dulbecco's Modified Eagle's Medium/Nutrient Mixture F-12 Ham (DMEM/F-12) (Merck Life Science, MO, USA) supplemented with an antioxidant, the 2-Mercaptoethanol (Thermo Fisher, MA, USA), and Insulin-Transferrin-Selenium (ITS-G) (Thermo Fisher, MA, USA). The two SFM differ for the addition of glutamine in SFM_B or the GlutaMAX^{™} Supplement (Thermo Fisher, MA, USA) in SFM_A.

### HEK-293T adaptation to serum-free medium

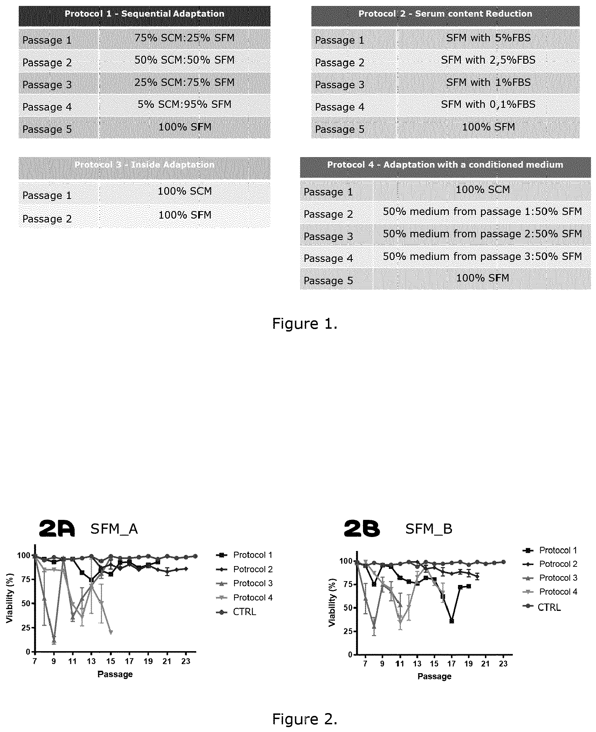

The HEK-293T cell culture is adapted to serum-free conditions through four different protocols (figure 1), commonly described in the literature [3]:
1. Sequential adaptation > cells are grown with a mixture of SCM and SFM. Progressively the SCM percentage is reduced whereas the SFM percentage is increased, until the complete serum removal;
2. Serum content Reduction > cells are cultured in SFM supplemented with a percentage of FBS that is decreased at each passage until the final concentration of 0%;
3. Inside adaptation > cells are directly seeded in SFM;
4. Adaptation with a conditioned medium > involves the use of a conditioned medium where the cells are growing for the previous passage mixed with the serum-free medium.

Cells are monitored with a microscope every 2-3 days and media exchanges are performed twice a week. Cells are moved to the next passage when the confluency is >70% and the viability >80%.

### Cell proliferation

Cell proliferation is analyzed in real-time using Incucyte^{®} (Sartorius). Incucyte^{®} is a real-time quantitative live-cell imaging and analysis platform that enable visualization and quantification of cell behavior over time by automatically gathering and analyzing images around the clock within a standard laboratory incubator.

At every split, 5x103 HEK-293T cells are plated in each well on a 6-well plate using the different serum-free adaptation protocols in SFM_A. Every hour, for a total of 4 days, images of every well are taken. For the analysis, a confluence mask is created to calculate the occupied area (% confluence) of cell images over time and generate cell growth curves.

### Cell viability during HEK 293-T adaptation in serum-free

One vial of HEK-293T cells is thawed and the cell viability is determined. Since no impairment of cell viability is observed, cells are divided into four protocols using two different SFM. Cell viability is measured at every split during the whole process and shown in figure 2.

With SFM_A the cells are successfully adapted to 100% serum-free with 3 out of 4 protocols maintaining high viability. Regarding, SFM_B cells are adapted serum-free only with protocol 2 in which cell viability >75% is observed. These results suggested that SFM_A support better HEK-293T cell adaptation in serum-free condition. This medium will be used for the following experiment.

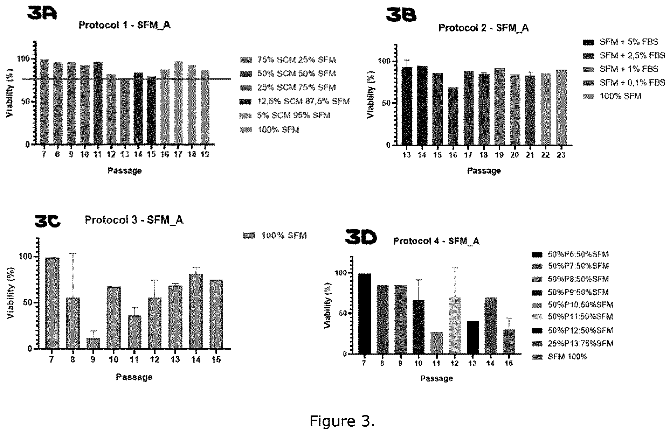

To identify the most promising process for HEK-293T cells adaptation in SFM_A, the viability obtained with the four protocols is analyzed in detail, and results are reported in Figure 3.

Protocols number 1 (3A) and 2 (3B) displayed cell viability >75% during the serum-free adaptation which is comparable with the one observed in the cell cultured in SCM used as control (CTRL) (Figure 2).

Protocol number 3 (3C) displayed an initial reduction in cell viability that is recovered starting from passage 11. Lastly, protocol 4 (3D), allowed the maintenance of high viability only for a few passages. For this reason, the protocol is discarded.

Based on the results previously obtained a new adaptation is performed using protocols 1, 2, and 3. To monitor cell morphology and confluency, cells are analyzed in real-time using Incucyte^{®} (Sartorius).

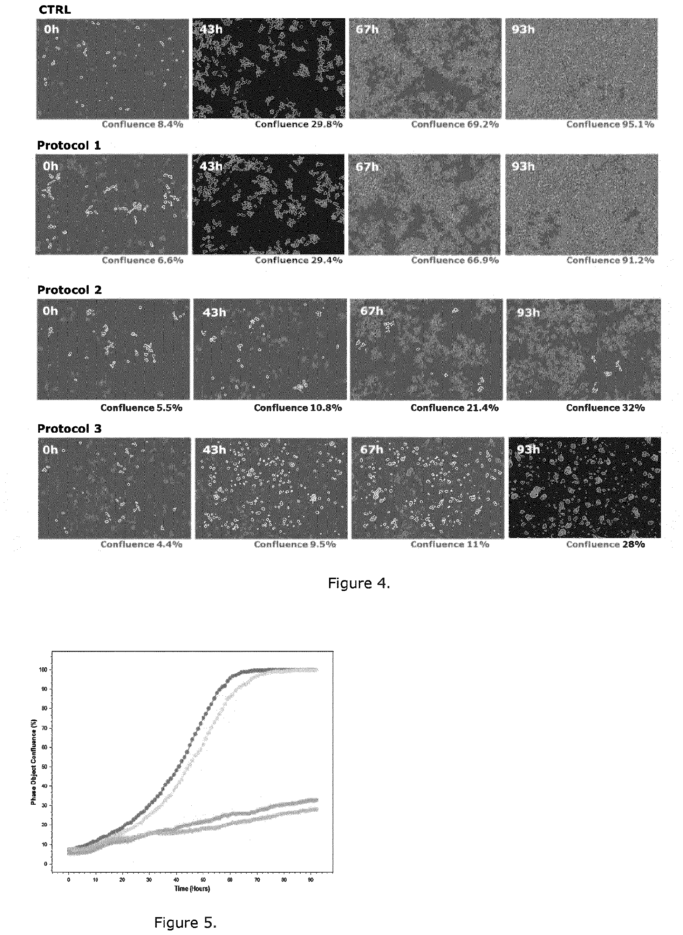

As shown in Figure 4, in protocols 1 and 2, HEK-293T cell morphology remain similar to the control one. However, with protocol 2 cell confluence after 93 hours is reduced compared to CTRL and protocol 1. On the other hand, in protocol 3, the cells have a smaller morphology and begin to stay in suspension and not in adhesion. This adaptation protocol affects cell growth. In fact, after 93 hours cell confluency reach 28%.

These observations are confirmed by the growth curves analysis which displayed a difference in cell proliferation among the protocols. As shown in Figure 5, HEK-293T cells in protocol 1 (yellow line) grew similarly compared to the CTRL (red line). The cells cultured with protocols 2 (green line) and 3 (blue line) displayed a strong slowdown in cell growth rate.

Thus, the HEK-293T cell line was adapted to grow in a serum-free medium. All the experimental conditions were tested and the medium SFM_A displayed the best performance in terms of cell viability. Regarding the different protocols, 3 out 4 showed cell viability >75% but only protocol 1 presented cell morphology and a growth rate curve similar to the control one.

The possibility to have a cell line adapted to grow in a serum-free environment could be an important resource for bioassay development. In fact, the serum-free cell line could be engineered to express a specific drug target with the final goal to develop a complete bioassay serum-free, increasing its reproducibility and reducing its variability.

### References

[1] International Pharceutical Quality. USP Chapter 1032. Design and development of Biological Assay. US Pharmacopeial Convention.
[2] J. V. D. VALK, "Fetal bovine serum-a cell culture dilemma," SCIENCE, vol. 375, pp. 143-144, 2022.
[3] B. D. e. a. Van der Valk J, "Optimization of chemically defined cell culture media - Replacing fetal bovine serum in mammalian in vitro methods," Toxicology in Vitro, pp. 1053 - 1063, 2010.
[4] E. J. H. K. J. v. L. J. W. H. V. J. v. Mandl, "Fibroblast growth factor-2 in serum-free medium is a potent mitogen and reduces dedifferentiation of human ear chondrocytes in monolayer culture," Matrix Biology, vol. 24, p. 231-241, 2004.
[5] T. M. B. R. e. a. Lomba A, "Serum-Free Suspension Adaptation of HEK-293T Cells: Basis for Large-Scale Biopharmaceutical Production," Brazilian Archives of Biology and Technology, 2021.

## Claims

1. A serum free medium comprising a) Dulbecco's Modified Eagle's Medium (DMEM), b) a nutrient mix, c) an antioxidant and d) a supplemental source of glutamine.

2. The serum free medium of claim 1, wherein the nutrient mix is nutrient mixture F-12 Ham.

3. The serum free medium of any of the preceding claims, wherein the antioxidant is 2-mercaptoethanol.

4. The serum free medium of any of the preceding claims, wherein the supplemental glutamine source is selected from glutamine or GlutaMAX.
